# EUROPEAN PATENT APPLICATION

(11) **EP 2 468 263 A1**
(43) Date of publication of application: **27.06.2012**
(21) Application number: 10382347.2
(22) Date of filing: 23.12.2010
(51) Int. Cl.: A61K 9/20, A61K 9/50

(54) **Compressed solid dosage forms**

(71) Applicant: Laboratorios del. Dr. Esteve, S.A., 08041 Barcelona (ES)
(72) Inventor: Soler Ranzani, Luis, E-8013 BARCELONA (ES); Falivene Aldea, Albert, E-8010 BARCELONA (ES); Santanach-Delisau, Ángel, E-8500 Vic- BARCELONA (ES); Casadevall Pujals, Gemma, E-8019 BARCELONA (ES)
(74) Representative: ABG Patentes, S.L.

(57) **Abstract**

This invention relates to compressed solid oral dosage form able to contain a high load of active pharmaceutical ingredient, showing a controlled release profile, and consisting of individual particles wherein each individual particle comprises an active pharmaceutical ingredient and wherein each individual particle is coated with a layer comprising at least one plasticizer.

## Description

### FIELD OF THE INVENTION

The present invention relates to new compressed solid dosage forms comprising at least one active pharmaceutical ingredient to be released in vivo. The invention further relates to new compressed solid dosage forms allowing a controlled release of the medicinal agent contained therein.

### BACKGROUND

Pharmaceutical dosage forms with different layers having different functions are widely used and described in the art. Furthermore, when said multi-layered dosage forms undergo a compression process, it has been described that functional properties of the layers are damaged. As a consequence, the active substance may be altered or even destroyed after in vivo absorption, and/or the liberation profile may be modified. This represents a great obstacle for formulations providing a non-immediate release.

The problem of ensuring a controlled, long-lasting, continuous effective action of a therapeutic agent in a human body has been addressed in different ways in the art, especially for compressed pharmaceutical dosage forms.

US-2005/0266078 describes orally dispersible multiparticulate tablets allowing a prolonged release of the active pharmaceutical ingredient, obtained starting from microcapsules developed for a therapeutic sustained release profile, by coating the particles with a protective layer absorbing the mechanical stresses due to compression. This allows to optimize the stability of the microparticle, and to maintain their initial properties after compression. The protective layer contains deformable organic constituents having a melting point between 40 °C and 120 °C. Such organic constituents are preferably polyethylene glycols having a molecular weight of 6000 to 20,000 D. These microparticles are then mixed with 70% of disintegrating agent, acting as a compression base, and compressed to give the sustained release tablets.

US-4,684,516 describes tablets composed of 85% to 98% of drug containing sustained release spherical pellets coated with one or more disintegrating agent such as starch, cellulose powder, cellulose ethers, polyacrylic acid, sodium alginate, alginic acid, modified cellulose gum and pectin.

WO-2009/043929 discloses a solid dosage form comprising controlled release pellets coated with a mechanical protective layer comprising a mixture of polyethylene glycols having a molecular weight of 4000, 6000 and 8000 D, and a compression base containing a mixture of lubricants, and surfactants.

WO-99/26608 discloses spheroids containing an active pharmaceutical ingredient, said spheroids being directly compressible, meaning without substantial addition of another auxiliary substance. In order to achieve this result, the described particles are coated with a flexible polymeric deformable material which glass transition temperature below 30°C. Nevertheless the result obtained when measuring the liberation profile shows an acceleration of the liberation of the active pharmaceutical ingredient in the compressed dosage forms, when compared to the spheroids before compression.

Further, sustained release characteristics of a drug are strongly influenced by their solubility and therefore the amount of sustained release agent needed to control drug release in the dosage form is increasing substantially. As a consequence, the suitability of sustained release agents to obtain a modified release tablet with a high drug loading could be compromised, particularly in the case of oral administration.

From the above, it is clear that the effect of the compression step in the manufacture of solid oral dosage forms may be of importance when the objective is to obtain a sustained release liberation of the active pharmaceutical ingredient.

Therefore, there is still a need to provide compressed solid oral dosage forms showing a modified release profile, in which it is possible to incorporate a high percentage of active pharmaceutical ingredient, and being easily manufactured. The amount of the sustained release agent or the mixture of several of said agents also need to be reduced.

### BRIEF DESCRIPTION OF THE INVENTION

The present invention proposes a simple technical solution to obtain compressed solid oral dosage forms able to contain a high load of active pharmaceutical ingredient and showing a controlled release profile.

In particular, the inventors of the present invention have surprisingly found a plastic deformable layer that can be sprayed directly onto particles. Said layer which coats the individual particles allows obtaining compressed solid oral dosage forms composed of 100% of said coated individual particles, without the need to use any other compression base or excipient as fillers, binders, disintegrants or lubricants. Therefore, one aspect of the present invention relates to a compressed solid oral dosage form consisting of individual particles wherein each individual particle comprises an active pharmaceutical ingredient and wherein each individual particle is coated with a layer comprising at least one plasticizer.

It has been found further that the compressed solid oral dosage form of the invention surprisingly shows modified release characteristics compared with the coated particles before compression which show an immediate release profile. Accordingly, a preferred embodiment of the invention is directed to such controlled release of the active pharmaceutical ingredient.

Another aspect of this invention refers to a compressed solid oral dosage form as defined above for use as a medicament.

Another aspect refers to a method of treatment of a medical condition comprising administering to a patient in need of such treatment a compressed solid oral dosage form as defined above comprising a therapeutically effective amount of the active pharmaceutical ingredient.

Another aspect of this invention refers to a process for the preparation of a compressed solid oral dosage form as defined above which comprises the steps of (i) coating individual particles with a layer comprising at least one plasticizer and (ii) compressing the obtained individual coated particles obtained in the previous step.

Another aspect of this invention refers to a compressed solid oral dosage form obtainable by a process as defined above.

Another aspect of this invention refers to a plastic layer as defined above for solid oral dosage forms. Preferably said plastic layer comprises a mixture of at least one polyethylenglycol with a molecular weight comprised between about 1000 and 20000, and at least another excipient.

These aspects and preferred embodiments thereof are additionally also defined in the claims.

### BRIEF DESCRIPTION OF THE FIGURES

Figure 1: Schematic general procedure for the preparation of tablets according to one embodiment of the present invention.
Figure 2: Schematic general procedure for the preparation of plastic film coating comprising PEG according to examples 1 to 5 of the present invention.
Figure 3: Schematic general procedure for the preparation of plastic film coating comprising PEG and fatty excipients according to examples 6 and 7 of the present invention.
Figure 4: Schematic general procedure for the preparation of plastic film coating comprising PEG and swelling polymers according to examples 8 and 9 of the present invention.
Figure 5: Schematic general procedure for the preparation of plastic film coating comprising PEG and insoluble excipients according to examples 10 and 11 of the present invention.
Figure 6: Comparative dissolution profile of tablets according to example 1 obtained with compressed and non-compressed pellets coated with about 5% of plastic layer.
Figure 7: Comparative dissolution profile of tablets according to example 2 obtained with compressed and non-compressed pellets coated with about 15% of plastic layer.
Figure 8: Comparative dissolution profile of tablets according to example 3 obtained with compressed and non-compressed pellets coated with about 25% of plastic layer.
Figure 9: Comparative dissolution profile of tablets according to example 4 obtained with compressed and non-compressed pellets coated with about 46% of plastic layer.
Figure 10: Comparative dissolution profile of tablets according to example 5 obtained with compressed and non-compressed pellets coated with about 59% of plastic layer.
Figure 11: Comparative release profile of tablets according to examples 1 to 5 obtained with an increasing amount of coating layer at pH 1.2 (figure 11a) and pH 5.5 (figure 11b).
Figure 12: Comparative in vitro release profiles of matrix tablets with a combination of PEG 4000 and compritol ATO888^{®} (Glyceryl dibehenate / tribehenin / glyceryl behenate) at coating levels of about 15 and about 46%, at pH 1.2 (figure 12a) and pH 5.5 (figure 12b).
Figure 13: Comparative in vitro release profiles of matrix tablets with a combination of PEG 4000 and chitosan at coating levels of about 15 and about 46%, at pH 1.2 (figure 13a) and pH 5.5 (figure 13b).
Figure 14: Comparative in vitro release profiles of matrix tablets with a combination of PEG 4000 and neusilin^{®} (magnesium aluminum metasilicate) at coating levels of about 15 and about 46%, at pH 1.2 (figure 14a) and pH 5.5 (figure 14b).
Figure 15: Comparative in vitro release profiles of matrix tablets with a combination of PEG and different excipients at coating levels of about 15 (figure 15a) and about 46% (figure 15b) at pH 1.2.

### DETAILED DESCRIPTION OF THE INVENTION

The present invention relates to compressed solid oral dosage forms based on individual particles wherein each individual particle comprises an active pharmaceutical ingredient and wherein each individual particle is coated with a layer comprising at least one plasticizer. Said individual particles are typically in form of multilayered pellets.

In comparison with the coated particles before compression which show an immediate release profile, the compressed solid oral dosage form of the invention surprisingly allows controlling the release of the active pharmaceutical ingredient. The term "individual particle" as used herein means discrete units of matter comprising the active pharmaceutical ingredient. These individual particles are later coated becoming "coated particles". Examples of particles include, but are not limited to, pellets, granules, mini-tablets and powder crystals.

As used herein, the term "controlled release" is intended to refer to any drug-containing formulation in which release of the drug is not immediate; i.e., with a "controlled release" formulation, oral administration does not result in immediate release of the drug into an absorption pool. The term is used interchangeably with "non-immediate release" or "modified release". The term "controlled release" as used herein is intended to include any non-immediate release formulation, such as sustained release formulations. The term "sustained release" is used in its conventional sense to refer to a drug formulation that provides for gradual release of drug over an extended period of time, and that preferably, although not necessarily, results in substantially constant blood levels of drug over an extended time period.

It is further remarkable in the present invention the lack of necessary sustained release agent to provide the required liberation profile, or alternatively if a sustained release agent is present, the amount used is not sufficient by itself to provide the sustained release effect.

Thanks in part to the low amount of sustained release agent, or to the total lack of the same, the compressed solid oral dosage forms according to the present invention make possible the incorporation of high doses of the active pharmaceutical ingredient in the formulation. In one embodiment, the amount of active pharmaceutical ingredient is greater than about 30%, preferably greater than about 50%, more preferably greater than about 75%, and even more preferably greater than about 85% of the total weight of the compressed dosage form. As the skilled person will appreciate, there is no real limitation in the present invention in terms of the active pharmaceutical ingredient. According to the invention, any active pharmaceutical ingredient for which a controlled release is desirable in order to achieve the better therapeutic effect, may be incorporated in the compressed solid oral dosage forms. In particular, active pharmaceutical ingredients for which the solubility in water is comprised between about 20 mg/ml and 600 mg/ml are preferred.

A compressed solid oral dosage form according to the present invention may contain more than one active pharmaceutical ingredient. In a particular variant, each individual particle comprises such combination of active pharmaceutical ingredients. In another particular variant, the dosage form consists of individual particles comprising at least one active pharmaceutical ingredient and individual particles comprising at least another different active pharmaceutical ingredient. Furthermore, the dosage form may contain a mixture of two or more populations of individual particles having different characteristics, such as different active pharmaceutical ingredient, size or coating. This fact may produce different release profiles of the different active pharmaceutical ingredients.

The core of the particles may preferably comprise excipients together to the active pharmaceutical ingredient. The skilled in the art will readily determine by routine experimentation the appropriate excipients depending upon the pharmaceutical active ingredient, the desired final dosage form, etc. The term "excipient" refers to a carrier, adjuvant or vehicle with which the pharmaceutical active ingredient is administered and include any such materials known in the art that are nontoxic and do not interact with other components of a pharmaceutical composition. Suitable pharmaceutical excipients are described in "Remington's Pharmaceutical Sciences" by E.W. Martin. For instance, in general, it is convenient to use fillers (diluents) to increase the bulk and reach a practical size for compression. Examples of fillers suitable as excipients include, but are not limited to, compressible sugar, dextrates, dextrin, dextrose, lactose, mannitol, sorbitol, microcrystalline cellulose, silicified microcrystalline cellulose, etc. The combination of above-mentioned fillers or other excipients can also be used. By way of example, a combination of PEG 8000 and MCC 102 is particularly preferred.

The individual particles of the invention are coated with a layer comprising at least one plasticizer. In one embodiment, the amount of the layer comprising at least one plasticizer is comprised between about 5 and 60% of weight with respect to the compressed dosage form weight. In another embodiment the amount of plastic layer is between about 5 and 30% or between about 30 and 60% by weight. In more particular embodiments the amount of the plastic layer is about 5, about 15, about 25, about 46 or about 59% of weight with respect to the compressed dosage form weight.

The term "plasticizer" refers to a substance that is habitually used to improve the physical and mechanical properties of a film, improving properties such as elastic elongation, deformability, tensile strength, flexibility. The expression "at least one plasticizer" is intended to include not only one plasticizer but a mixture of plasticizers such as two, three or four plasticizers. Further, as will be defined below, in addition to the plasticizer or plasticizers, another excipients may be preferably present in the layer.

In a particular embodiment the at least one plasticizer is selected from polyethylenglycol (PEG), short, medium and long chain fatty acids, triglyceride fatty acids, wax, fatty alcohols, gliceryl monoestearate, glycerol palmito stearate, triethyl citrate, dibutyl sebacate, acetyl tributyl citrate. Preferably, the plasticizer is polyethylenglycol. More preferably, the plasticizer is polyethylenglycol with a molecular weight comprised between about 1000 and about 20000, and more preferably between about 1000 and about 12000. In particular embodiments the molecular weight of the polyethylenglycol is about 4000, about 6000 or about 8000.

According to a preferred variant of the invention, the layer comprises at least one plasticizer, preferably polyethylenglycol, and at least another excipient. In preferred embodiments, the plastic layer is a mixture of at least one polyethylenglycol with a molecular weight comprised between about 1000 and about 20000 and at least another excipient. More preferably, the plastic layer is a mixture of at least one polyethylenglycol with a molecular weight comprised between about 1000 and 12000, and at least another excipient.

The other excipients that may be used according to the present invention in the plastic layer are selected among swelling polymers such as glucopyranosamine polymer, glucosamine-glucopyranosamine co-polymers (e.g. Chitosan); polyethylenoxydes; alginates; polycarbophil, carbomer, poloxamer; cellulose polymers such as hydroxypropylmethylcellulose, hydroxypropylcellulose, hydroxyethylcellulose, hydroxyethylmethylcellulose; fatty acids (e.g. stearic acid); fatty esters (e.g. Glyceryl dibehenate / tribehenin / glyceryl behenate = COMPRITOL 888®); fatty alcohols (e.g. cetyl alcohol); and other insoluble excipients such as magnesium aluminium metasilicate, microcrystalline cellulose or calcium hydrogen phosphate dehydrate, poly(lactic-co-glycolic acid). In a preferred embodiment, none of the excipients present in the plastic layer confer controlled release properties, i.e. either there is no controlled release agent or, if it is present, its amount is not enough to provide said properties.

In a further embodiment the plasticizer or plasticizers, particularly PEG, represent at least about 60% of the plastic layer by weight, preferably at least about 75%, and more preferably at least 85%. In another embodiment, the plasticizer(s) such as PEG represents between about 60 and about 90% of the plastic layer by weight.

In a yet preferred embodiment, the plastic layer comprises at least one plasticizer, at least one lubricant, and at least another excipient. More particularly, the at least one plasticizer is polyetylenglycol and the at least one lubricant is preferably glyceryl monostearate (GMS). In particular embodiments, the glyceryl monostearate is present in a range from 0.5% to 5.8% of the weight of the final dosage form.

It is clear that a compressed solid oral dosage form according to the present invention may contain particles coated by different plastic layers as defined herein.

The process for the preparation of the compressed solid oral dosage form of the invention may be carried out following methods widely-known in the art (see "Remington, the Science and practice of pharmacy", 21 st Edition, 2005, Ed. Lippincott Williams & Wilkins). The process for manufacture is simple as it uses conventional equipment and a limited number of process steps.

In a first step (i), the process comprises coating the individual particles with a layer comprising at least one plasticizer. The plastic layer may be easily applied using well-known methods for the skilled person; for example, it may be sprayed directly onto particles. Advantageously, the process may be carried out starting from available conventional particles since a large range of particle size, from 0.04 µm to 1200 µm, can be processed according to the present invention to form the compressed oral solid dosage form. Preferred particles in this invention are pellets. A review of the different methods for obtaining pellets for pharmaceutical purposes can be found for example in the book Pharmaceutical Pelletization Technology, edited by Isaac Ghebre-Sellassie, Marcel Dekker, Inc., 1989.

In a second step (ii), the process comprises compressing the obtained individual coated particles obtained in the previous step. The term "compressing" is intended to include any known process performed by applying compression forces and optionally heating. These methods include, but are not limited to, compression, compaction, extrusion and injection moulding. In a preferred embodiment the method used is compression. The compression is typically performed under compression forces from 1000 to 40000 N, preferably from 5000 to 20000 N, and more preferably from 10000 to 12000 N. Direct compression is the easiest way of manufacturing tablets as there is no need of intermediate step(s) as blending or granulation before the compression step. The cost of manufacturing is thus considerably reduced.

A further aspect of the invention concerns a compressed solid oral dosage form obtained by a process as defined above. The resulting compressed solid formulation possesses a suitable strength and hardness and do not disintegrate during distribution and storage.

According to a preferred embodiment the compressed solid oral dosage form is a tablet. Further, such a tablet may be coated after being pressed to afford for example a sugar-coated tablet or a film-coated tablet.

The present invention is also directed to the plastic layer as defined above for solid oral dosage forms in itself. Preferably said plastic layer comprises a mixture of at least one polyethylenglycol with a molecular weight comprised between about 1000 and 20000 and at least another excipient. More preferably, the plastic layer comprises a mixture of at least one polyethylenglycol with a molecular weight comprised between about 1000 and 12000, and at least another excipient.

As used herein, the term "about" means a slight variation of the value specified, preferably within 10 percent of the value specified. Nevertheless, the term "about" can mean a higher tolerance of variation depending on for instance the experimental technique used. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

Unless otherwise stated, all amounts are expressed as percentage by weight.

The following examples are merely illustrative of certain embodiments of the invention and cannot be considered as restricting it in any way.

### EXAMPLES

### Solubility model for drugs:

The active pharmaceutical ingredient (API) "A" used to illustrate the present invention has been selected for its physicochemical properties. It has a pKa of 6.8, being a weakly basic drug and its solubility is strongly influenced by the pH of the medium. The solubility of the active pharmaceutical ingredient is decreasing when is increasing the pH of the medium. At acidic pH, the active pharmaceutical ingredient is highly soluble, 600 mg/ml for a pH of 1.2, while at a higher pH of 5.5 the solubility is 20 mg/ml. This allows demonstrating the utility of the present invention for different type of active pharmaceutical ingredients of different solubility.

### Examples 1 to 5:

A mixture of immediate release pellets of active pharmaceutical ingredient·HCl was coated with a PEG equiv ratio of 6467 at different coating levels from about 5 to about 59%, according to the following general procedure:

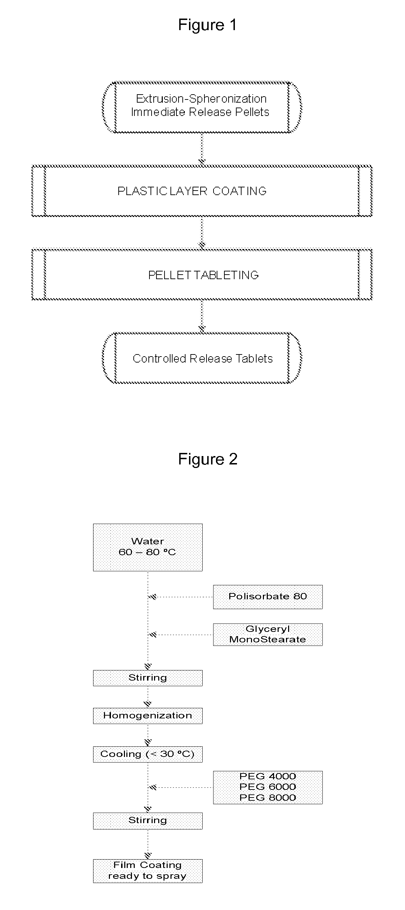

### Preparation of the PEG based layer (see figure 2):

Polisorbate 80 was added to water heated at a temperature of 60 to 80 °C under helix stirring. Glyceryl monostearate was added to the obtained mixture under helix stirring and agitation was maintained until a homogeneous mixture was obtained, followed by homogenization using an ultraturrax homogenizer.

After homogenization, the dispersion was cooled down to a temperature below 30 °C. The PEG (PEG 4000, PEG 6000 and/or PEG 8000) was added to the cold dispersion, under helix stirring and was dissolved. The film coating was filtered through a 0.5 mm sieve.

### General procedure for the preparation of the tablets according to examples 1 to 5:

The obtained dispersion was sprayed on the core pellets in fluid bed coater.

The coated individual particles are directly compressed to achieve a 600 mg tablet with a tabletting machine using a compression force of 10000 to 12000 N.

The compositions of the tablets obtained accordingly are the following:
Composition table, example 1:

| **5 % PEG eq 6467 based Coating** | **Ingredient** | **Tablet** | |
|---|---|---|---|
| | | mg | % |
| **Core** | **API** | **490.34** | **81.72** |
| | MCC 102 | 59.45 | 9.91 |
| | PEG 8000 | 16.42 | 2.74 |
| | | | |
| **Layer** | PEG 4000 | 8.53 | 1.42 |
| | PEG 6000 | 6.07 | 1.01 |
| | PEG 8000 | 15.66 | 2.61 |
| | Glyceryl Monostearate | 3.28 | 0.55 |
| | Polisorbate 80 | 0.25 | 0.04 |
| **TOTAL** | | **600.0** | **100.0** |

Composition table, example 2:

| **15 % PEG eq 6467 based Coating** | **Ingredient** | **Tablet** | |
|---|---|---|---|
| | | mg | % |
| **Core** | **API** | **441.24** | **73.54** |
| | MCC 102 | 53.50 | 8.92 |
| | PEG 8000 | 14.78 | 2.46 |
| | | | |
| **Layer** | PEG 4000 | 22.85 | 3.81 |
| | PEG 6000 | 16.26 | 2.71 |
| | PEG 8000 | 41.93 | 6.99 |
| | Glyceryl Monostearate | 8.79 | 1.46 |
| | Polisorbate 80 | 0.66 | 0.11 |
| **TOTAL** | | **600.0** | **100.0** |

Composition table, example 3:

| **25 % PEG eq 6467 based Coating** | **Ingredient** | **Tablet** | |
|---|---|---|---|
| | | mg | % |
| **Core** | **API** | **388.7** | **64.8** |
| | MCC 102 | 47.1 | 7.9 |
| | PEG 8000 | 13.0 | 2.2 |
| | | | |
| **Layer** | PEG 4000 | 38.2 | 6.4 |
| | PEG 6000 | 27.1 | 4.5 |
| | PEG 8000 | 70.0 | 11.7 |
| | Glyceryl Monostearate | 14.7 | 2.4 |
| | Polisorbate 80 | 1.1 | 0.2 |
| **TOTAL** | | **600.0** | **100.0** |

Composition table, example 4:

| **46 % PEG eq 6467 based Coating** | **Ingredient** | **Tablet** | |
|---|---|---|---|
| | | mg | % |
| **Core** | **API** | **278** | **46.3** |
| | MCC 102 | 34 | 5.7 |
| | PEG 8000 | 9 | 1.5 |
| | | | |
| **Layer** | PEG 4000 | 71 | 11.8 |
| | PEG 6000 | 50 | 8.3 |
| | PEG 8000 | 129 | 21.5 |
| | Glyceryl Monostearate | 27 | 4.5 |
| | Polisorbate 80 | 2 | 0.3 |
| **TOTAL** | | **600.0** | **100.0** |

Composition table, example 5:

| **59 % PEG eq 6467 based Coating** | **Ingredient** | **Tablet** | |
|---|---|---|---|
| | | mg | % |
| **Core** | **API** | **209.5** | **34.9** |
| | MCC 102 | 25.4 | 4.2 |
| | PEG 8000 | 7.0 | 1.2 |
| | | | |
| **Layer** | PEG 4000 | 90.4 | 15.1 |
| | PEG 6000 | 64.3 | 10.7 |
| | PEG 8000 | 165.9 | 27.7 |
| | Glyceryl Monostearate | 34.8 | 5.8 |
| | Polisorbate 80 | 2.6 | 0.4 |
| **TOTAL** | | **600.0** | **100.0** |

### Examples 6 and 7:

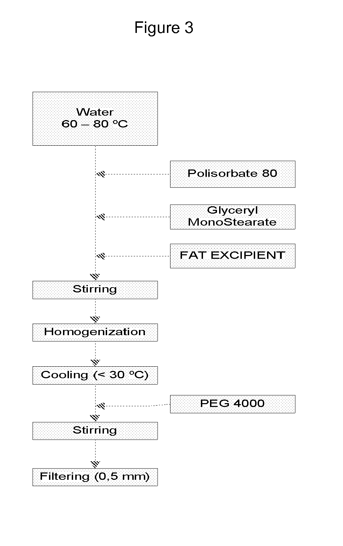

### Preparation of the PEG based layer containing fat excipients (see figure 3):

Polisorbate 80 was added to water heated at a temperature of 60 - 80 °C under helix stirring. Glyceryl monostearate was added to the obtained mixture, under helix stirring, and after its dispersion, the fat excipient, glyceryl dibehenate (Compritol ATO 888®), was added under helix stirring. The dispersion was stirred until homogeneous, and then further homogenized using an ultraturrax homogenizer.

After homogenization, the dispersion was cooled down to a temperature below 30 °C. The PEG 4000 was added to the cold dispersion under helix stirring and dissolved. The film coating was filtered through a 0.5 mm sieve.

### General procedure for the preparation of the tablets according to examples 6 and 7:

The obtained dispersion was sprayed to the core pellets in fluid bed coater.

The coated individual particles were directly compressed to achieve a 600 mg tablet with a tabletting machine using compression force of 10000 to 12000 N.
Composition table, example 6:

| **15 % PEG and Fat Excipients based Coating** | **Ingredient** | **Tablet** | |
|---|---|---|---|
| | | mg | % |
| **Core Pellets** | **API** | 437.73 | 72.96 |
| | MCC 102 | 53.07 | 8.85 |
| | PEG 8000 | 14.66 | 2.44 |
| | | | |
| **Layer** | PEG 4000 | 63.08 | 10.51 |
| | Glyceryl Monostearate | 11.18 | 1.86 |
| | Polisorbate 80 | 0.84 | 0.14 |
| | Glyceryl Behenate | 19.44 | 3.24 |
| **TOTAL** | | **600.0** | **100.0** |

Composition table, example 7:

| **46 % PEG and Fat Excipients based Coating** | **Ingredient** | **Tablet** | |
|---|---|---|---|
| | | mg | % |
| **Core Pellets** | **API** | 277.53 | 46.25 |
| | MCC 102 | 33.65 | 5.61 |
| | PEG 8000 | 9.29 | 1.55 |
| | | | |
| **Layer** | PEG 4000 | 186.52 | 31.09 |
| | Glyceryl | 33.05 | 5.51 |
| | Monostearate | | |
| | Polisorbate 80 | 2.47 | 0.41 |
| | Glyceryl Behenate | 57.48 | 9.58 |
| **TOTAL** | | **600.0** | **100.0** |

### Examples 8 and 9:

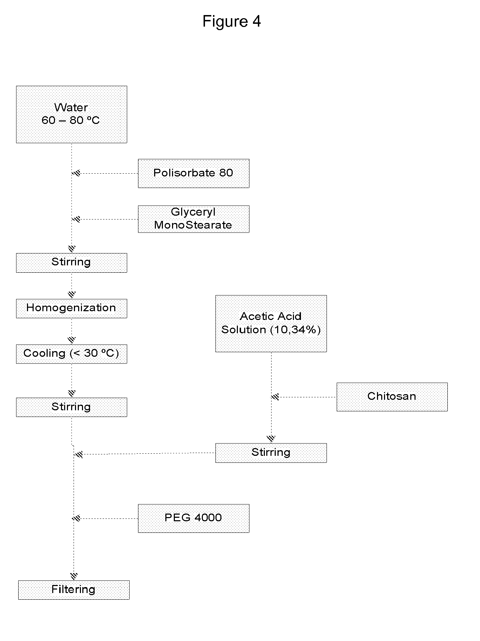

### Preparation of the PEG based layer containing swelling excipients (chitosan) (see figure 4):

Polisorbate 80 was added to water heated to a temperature of 60 - 80 °C under helix stirring. Glyceryl monostearate was added under helix stirring to the obtained solution.

The resulting dispersion was stirred until homogeneous, and then further homogenized using an ultraturrax homogenizer.

The obtained dispersion was cooled down to a temperature below 30 °C, and the chitosan, previously dissolved in a 10.34 % acetic acid solution, was added. PEG 4000 was then added under helix stirring and dissolved. The obtained film coating dispersion was filtered through a 0.5 mm sieve.

### General procedure for the preparation of the tablets according to examples 8 and 9:

The obtained dispersion was sprayed to the core pellets in fluid bed coater.

The coated individual particles were directly compressed to achieve a 600 mg tablet with a tabletting machine using a compression force of 10000 to 12000 N.
Composition table, example 8:

| **15 % PEG and Swelling Excipients based Coating** | **Ingredient** | **Tablet** | |
|---|---|---|---|
| | | mg | % |
| **Core** | **API** | 441.53 | 73.62 |
| | MCC 102 | 53.53 | 8.93 |
| | PEG 8000 | 14.79 | 2.47 |
| | | | |
| **Layer** | PEG 4000 | 67.53 | 11.38 |
| | Glyceryl Monostearate | 11.95 | 1.73 |
| | Polisorbate 80 | 0.95 | 0.13 |
| | Chitosan PM | 9.72 | 1.75 |
| **TOTAL** | | **600.0** | **100.0** |

Composition table, example 9:

| **46 % PEG and Swelling Excipients based Coating** | **Ingredient** | **Tablet** | |
|---|---|---|---|
| | | mg | % |
| **Core** | **API** | 285.65 | 47.61 |
| | MCC 102 | 34.63 | 5.77 |
| | PEG 8000 | 9.57 | 1.59 |
| | | | |
| **Layer** | PEG 4000 | 202.36 | 33.73 |
| | Glyceryl Monostearate | 35.82 | 5.97 |
| | Polisorbate 80 | 2.83 | 0.47 |
| | Chitosan PM | 29.14 | 4.86 |
| **TOTAL** | | **600.0** | **100.0** |

### Examples 10 and 11 :

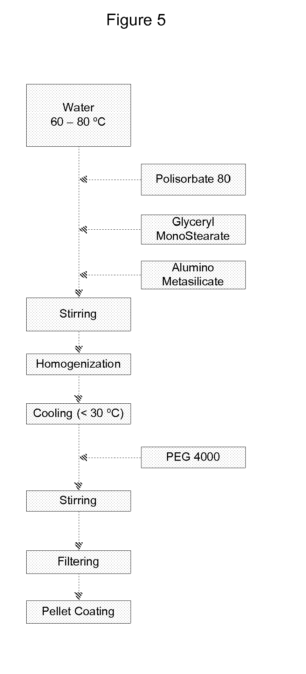

### Preparation of the PEG based layer containing insoluble excipients (Magnesium Aluminum silicate (Neusilin^{®})(see figure 5):

Polisorbate 80 was added to water heated to a temperature of 60 - 80 °C under helix stirring. Glyceryl monostearate was added to the resulting solution under helix stirring, followed by the addition of Neusilin^{®}.

After homogenization, the dispersion was cooled down to a temperature below 30 °C, and PEG 4000 was added under helix stirring. After complete dissolution of the PEG 4000, the film coating was filtered through a 0.5 mm sieve.

### Preparation of the tablets according to examples 10 and 11:

The obtained film coating dispersion was sprayed to the core pellets in a fluid bed coater.

The coated pellets are directly compressed to achieve a 600 mg tablet with a single punch korch tabletting machine using a compression force of 10000 to 12000 N.
Composition table, example 10:

| **15 % PEG and Insoluble Excipients based Coating** | **Ingredient** | **Tablet** | |
|---|---|---|---|
| | | mg | % |
| **Core** | **API** | 441.73 | 73.62 |
| | MCC 102 | 53.56 | 8.93 |
| | PEG 8000 | 14.79 | 2.47 |
| | | | |
| **Layer** | PEG 4000 | 68.25 | 11.38 |
| | Glyceryl Monostearate | 10.36 | 1.73 |
| | Polisorbate 80 | 0.79 | 0.13 |
| | Neusilin | 10.52 | 1.75 |
| **TOTAL** | | **600.0** | **100.0** |

Composition table, example 11:

| **46 % PEG and Insoluble Excipients based Coating** | **Ingredient** | **Tablet** | |
|---|---|---|---|
| | | mg | % |
| **Core** | **API** | 280.76 | 46.79 |
| | MCC 102 | 34.04 | 5.67 |
| | PEG 8000 | 9.40 | 1.57 |
| | | | |
| **Layer** | PEG 4000 | 209.35 | 34.89 |
| | Glyceryl Monostearate | 31.77 | 5.30 |
| | Polisorbate 80 | 2.42 | 0.40 |
| | Neusilin | 32.26 | 5.38 |
| **TOTAL** | | **600.0** | **100.0** |

### Example 12: Comparative dissolution profile of tablets according to examples 1 to 5

The dissolution profile of the tablets prepared according to examples 1 to 5 was obtained according to the general procedure using 900 ml of a 0.1 N HCl solution at pH 1.2, stirring at 50 rpm. They have been compared to the dissolution profile of the corresponding pellets without being compressed.

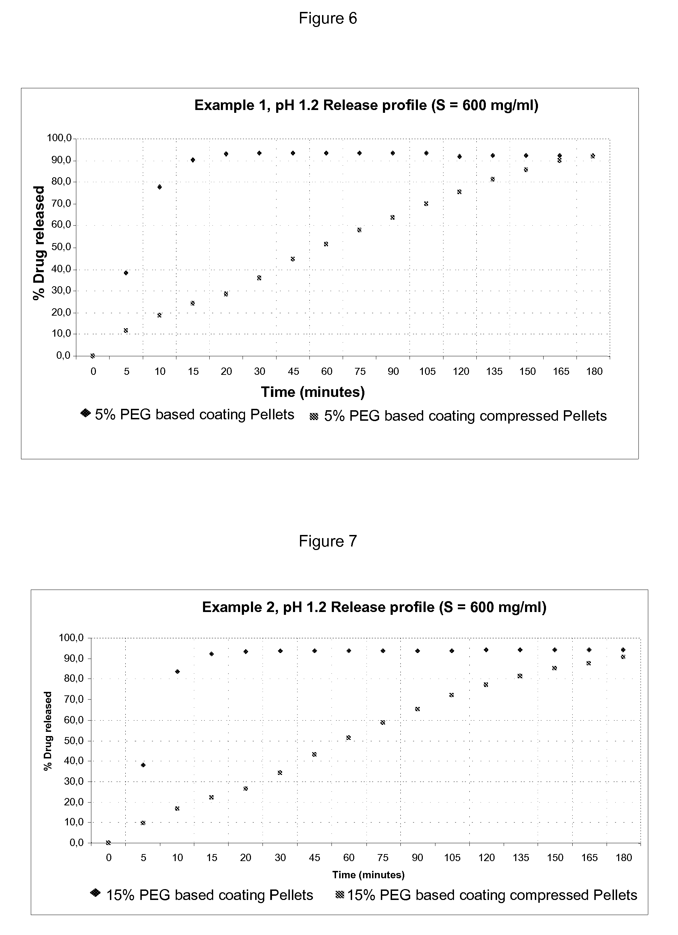

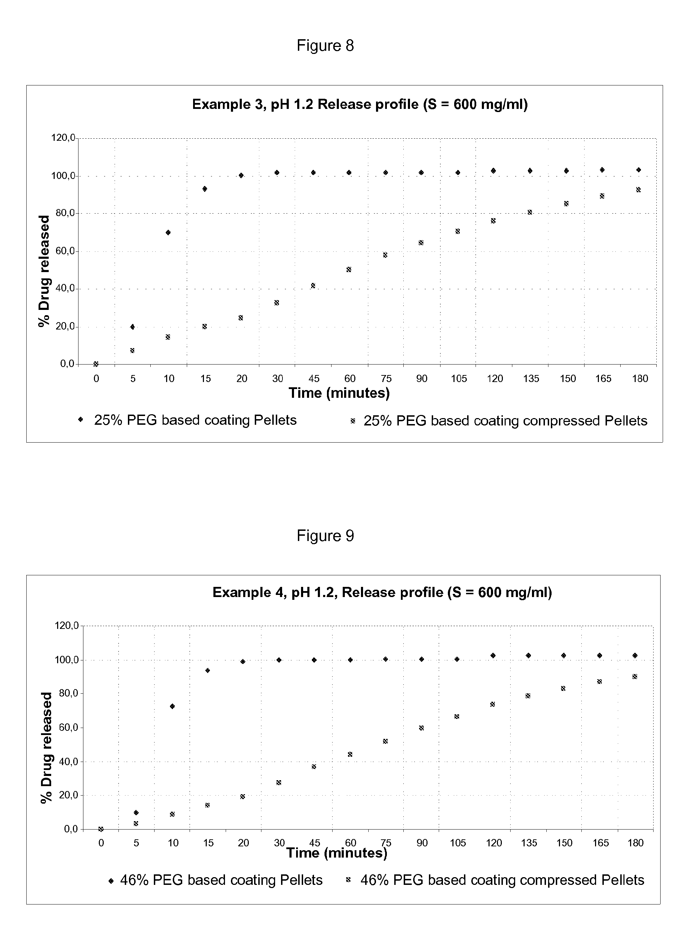

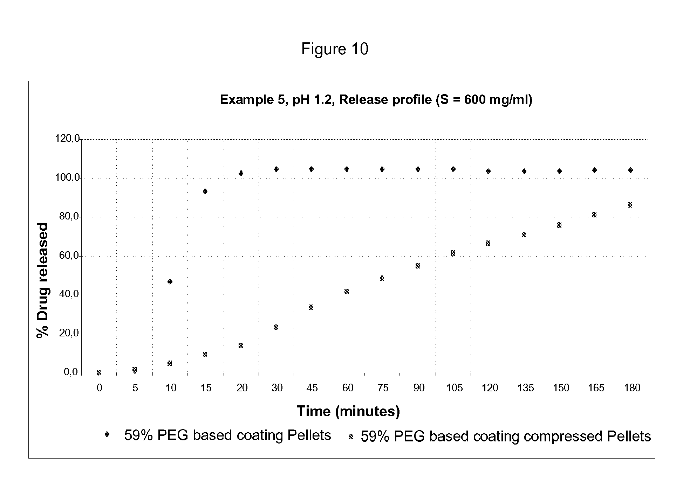

The results shown in figures 6 to 10 clearly demonstrate that the dissolution rate of the active pharmaceutical ingredient is delayed when the PEG coated pellets are compressed, whereas for the pellets without compression, the dissolution is achieved after 5 to 10 minutes.

### General procedure for drug release profile studies:

In vitro drug release studies were carried out in USP apparatus II paddle method. The drug release studies were undertaken at 37 °C ± 0.5 °C in two different medias, HCl 0.1 N, pH 1.2, and buffer phosphate solution pH 5.5 . The volume of dissolution was 900 ml, and the paddle speed 50 rpm. At regular time intervals samples were extracted and replaced with fresh medium. The samples were filtered through 0.45 µm sieve and assayed spectrophotometrically at 254 nm for compound A.

### Example 13: Comparative release profile of tablets according to examples 1 to 5

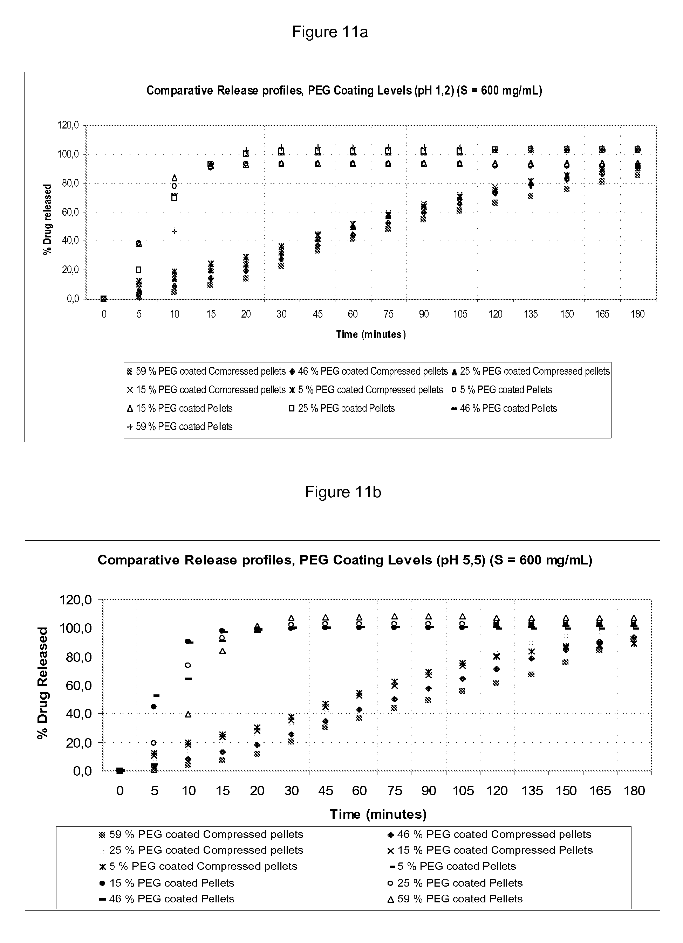

The release profiles of the active pharmaceutical ingredient contained in the tablets prepared according to examples 1 to 5 were obtained according to the general procedure using a solution at pH 1.2, 0.1N HCl, 50 rpm and 900 ml (figure 11a), and using buffer solution at pH 5.5, 50 rpm and 900 ml (figure 11b). They were further compared to the release profile of the active pharmaceutical ingredient contained in the corresponding pellets without compressing.

The results clearly show that the release of the active ingredient is delayed when the amount of PEG in the plastic layer is increased. Furthermore, it is also demonstrated that the individual particles when they are used without compressing step showed an immediate release profile.

### Example 14: Comparative release profile of tablets according to examples 6 and 7

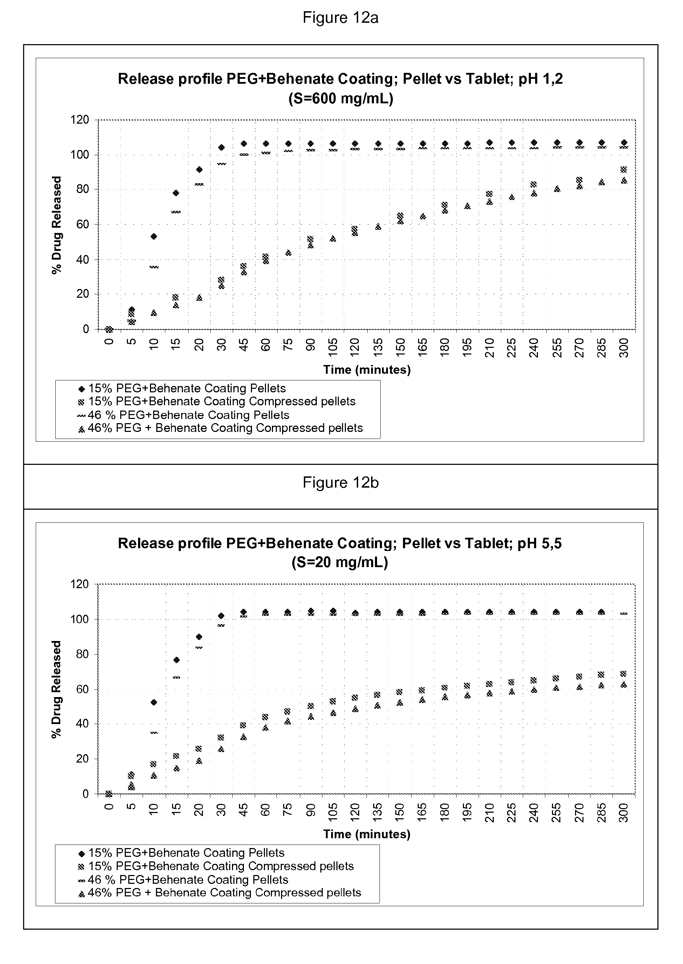

The release profiles of the active pharmaceutical ingredient contained in the tablets prepared according to examples 6 (about 15% coating) and 7 (about 46% coating) were obtained according to the general procedure, using a solution at pH 1.2, HCI 0.1 N, 50 rpm and 900 ml (Figure 12a), and using buffer solution at pH 5.5, 50 rpm and 900 ml (figure 12b.).

### Results:

Coated pellets at about 15% and about 46% performed as a control showed an immediate release profile: >80% drug is dissolved at 20 minutes.

Compressed solid oral dosage forms according to examples 6 and 7 of the present invention, exhibit sustained release characteristics: approximately 80% of drug release is achieved at 240 minutes in HCl 0.1 N wherein the active pharmaceutical ingredient A is highly soluble (600 mg/ml). In buffer solution at pH 5.5, wherein the solubility of the active pharmaceutical ingredient A is 20 mg/ml, the 80% of drug release is obtained up to 500 minutes.

It has been observed a similar release profile of matrix tablets obtained at different coating levels than 15 and 46%.

### Example 15: Comparative release profile of tablets according to example 8 and 9

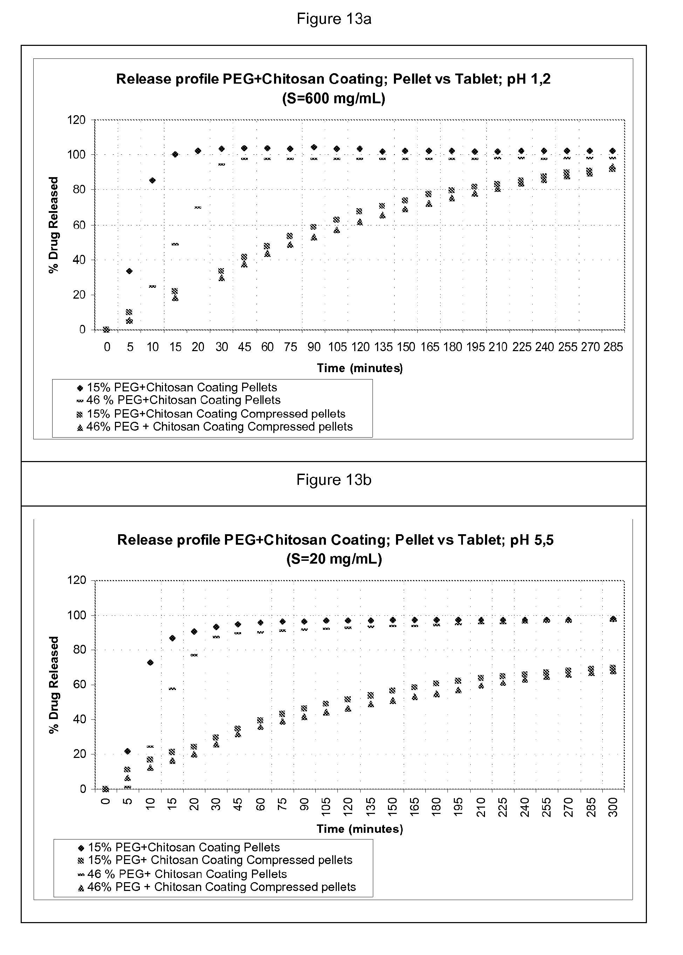

The release profiles of the active pharmaceutical ingredient contained in the tablets prepared according to examples 8 (about 15% coating) and 9 (about 46% coating) were obtained according to the general procedure, using a solution at pH 1.2, 0.1 N HCl, 50 rpm and 900 ml (figure 13a), and using buffer solution at pH 5.5, 50 rpm and 900 ml (figure 13b).

### Results:

Coated pellets at about 15% and about 46% showed an immediate release profile: >80% drug is dissolved at 30 minutes.

Compressed solid oral dosage forms according to example 8 and 9 of the present invention exhibit sustained release characteristics: approximately 80% of drug release is achieved at 180 minutes in HCl 0.1 N wherein active pharmaceutical ingredient is highly soluble (600 mg/ml), and in buffer solution pH 5.5 where the active pharmaceutical ingredient is soluble (20 mg/ml), the 80% of drug release is obtained up to 500 minutes.

### Example 16: Comparative release profile of tablets according to examples 10 and 11

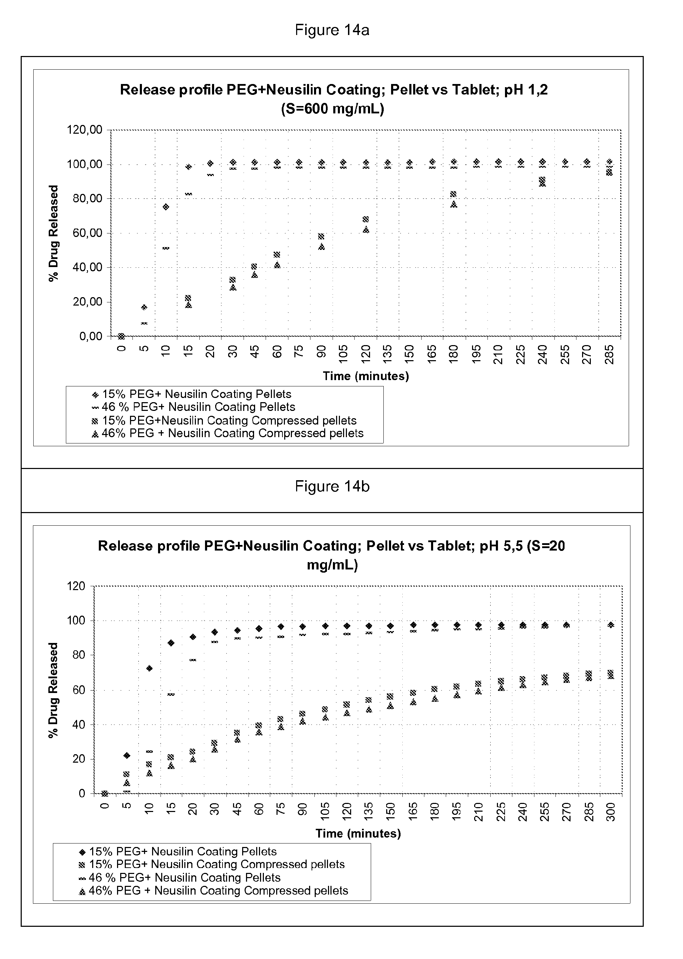

Figure 14 shows comparative in vitro release profiles of matrix tablets with a combination of PEG 4000 and Magnesium aluminum metasilicate at coating levels of about 15 and about 46%.

The release profiles of the active pharmaceutical ingredient contained in the tablets prepared according to examples 10 (about 15% coating) and 11 (about 46% coating) were obtained according to the general procedure, using a solution at pH 1.2, 0.1 N HCl, 50 rpm and 900 ml (figure 14a), and using solution at pH 5.5, 0.1 N HCl, 50 rpm and 900 ml (figure 14b).

### Results:

Coated pellets at about 15% and about 46% showed an immediate release profile: >85% drug is delivered at 15 minutes.

Compressed solid oral dosage forms according to example 10 and 11 of the present invention exhibit sustained release characteristics: approximately 80% of drug release is achieved at 150 minutes in HCl 0.1 N wherein the active pharmaceutical ingredient is highly soluble (600 mg/ml), and in buffer solution pH 5.5 where the active pharmaceutical ingredient is soluble (20 mg/ml), the 80% of drug release is obtained up to 500 minutes.

### Example 17: Comparative release profile of tablets according to examples 2, 4

### and 6 to 11

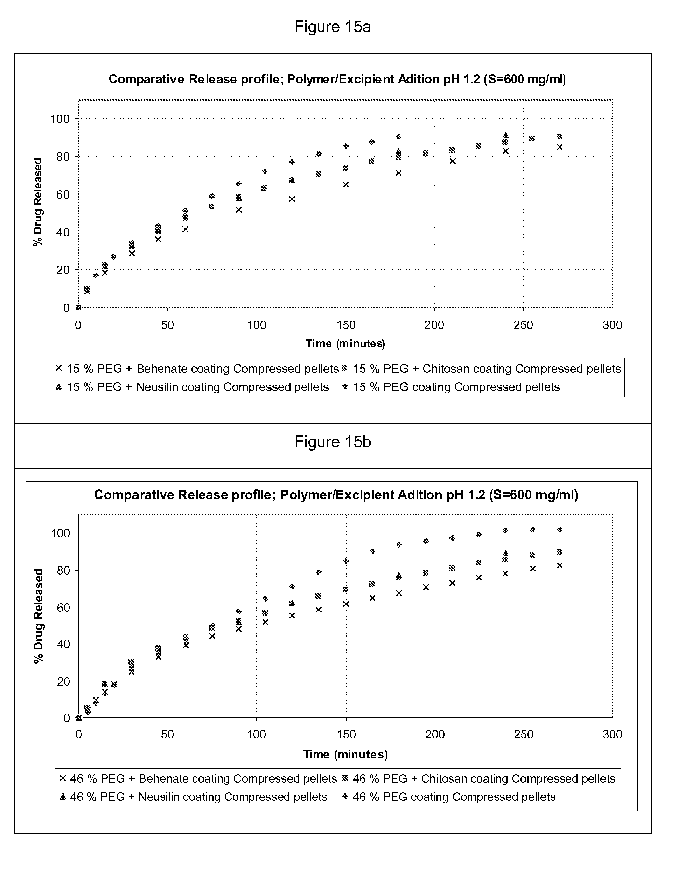

Figure 15 shows comparative in vitro release profiles of matrix tablets with a combination of different excipients at the layer composition at coating levels of about 15 and about 46%.

The release profiles of the active pharmaceutical ingredient contained in the tablets prepared according to examples 2, 6, 8, and 10 (about 15% coating) (figure 15a) and 4, 7, 9, and 11 (about 46% coating) (figure 15b) were obtained according to the general procedure, using solution at pH 1.2, HCl 0.1 N, 50 rpm and 900 ml.

### Results:

The comparison of the compressed solid oral dosage forms according to examples 2, 4 and 6 to 11 show the effect of the different polymers and excipients on the sustained release characteristics of the tablets, and further demonstrate that all these excipients are equivalent to solve the problem on which the present invention is based.

## Claims

1. A compressed solid oral dosage form consisting of individual particles wherein each individual particle comprises an active pharmaceutical ingredient and wherein each individual particle is coated with a layer comprising at least one plasticizer.

2. The dosage form according to claim 1 which provides a controlled release of the active pharmaceutical ingredient.

3. The dosage form according to claims 1 or 2, wherein the amount of layer is between about 5 and 60% of weight with respect to the dosage form weight.

4. The dosage form according to any of the preceding claims, wherein the at least one plasticizer represents at least about 60% of the layer's weight, preferably at least about 85% of the layer's weight.

5. The dosage form according to any of the preceding claims, wherein the at least one plasticizer is polyethylenglycol, preferably polyethylenglycol having a molecular weight comprised between about 1000 and 20000, more preferably between about 1000 and 12000.

6. The dosage form according to any of the preceding claims, wherein the layer further comprises at least another excipient.

7. The dosage form according to any of claims 6, wherein the at least another excipient is selected from the group consisting of swelling polymers, such as glucopyranosamine polymer, glucosamine - glucopyranosamine co-polymers; polyethylenoxydes; alginates; polycarbophil, carbomer, poloxamer; cellulose polymers, such as hydroxypropylmethylcellulose, hydroxypropylcellulose, hydroxyethylcellulose; hydroxylethylmethylcellulose, fatty acids; fatty esters; fatty alcohols; and other insoluble excipients, such as magnesium aluminum metasilicate, microcrystalline cellulose or calcium hydrogen phosphate dehydrate, poly(lactic-co-glycolic acid).

8. The dosage form according to any of the preceding claims, wherein the layer comprises polyethylenglycol having a molecular weight comprised between about 1000 and 12000 and at least another excipient as defined in claim 7.

9. The dosage form according to any of the preceding claims, wherein the amount of active pharmaceutical ingredient is greater than about 50% of the dosage form's weight, preferably greater than about 75%, more preferably greater than about 85%.

10. The dosage form according to any of the preceding claims **characterised in that** it is a tablet.

11. A compressed solid oral dosage form as defined in any of the preceding claims for use as medicament.

12. A process for the preparation of a compressed solid oral dosage form as defined in any of claims 1 to 11, said process comprising the steps of:
(i) coating individual particles with a layer comprising at least one plasticizer, and
(ii) compressing the obtained individual coated particles obtained in the previous step.

13. A compressed solid oral dosage form obtainable by a process as defined in claim 12 in form of a tablet.

14. A layer for solid oral dosage form comprising a mixture of at least one polyethylenglycol with a molecular weight comprised between about 1000 and 20000 and at least another excipient, preferably selected from the group defined in claim 7.

15. The layer according to claim 14, wherein the polyethylenglycol has a molecular weight comprised between about 1000 and 12000 and represents at least 60% of the layer, preferably represents at least 85% of the layer.
